Europäisches Patentamt

European Patent Office

Office européen des brevets

(11) Publication number: **0 192 298**
**A1**

## (12) EUROPEAN PATENT APPLICATION

(21) Application number: 86200208.6

(22) Date of filing: **14.02.86**

(51) Int. Cl.⁴: **C07C 45/58** , C07C 49/403 , C07C 49/08 , B01J 23/40

(30) Priority: 15.02.85 NL 8500430

(43) Date of publication of application:
27.08.86 Bulletin 86/35

(84) Designated Contracting States:
AT BE CH DE FR GB IT LI NL

(71) Applicant: STAMICARBON B.V.
Mijnweg 1
NL-6167 AC Geleen(NL)

(72) Inventor: Van Geem, Paul Christiaan
Constantijn Hugostraat 13
NL-6176 BS Spaubeek(NL)
Inventor: Janssen, Ludovicus Hubertus Wilhelmus
Henri Hermanslaan 51
NL-6162 GA Geleen(NL)

(74) Representative: Roeffen, Wilhelmus Johannes Maria
et al
OCTROOIBUREAU DSM Postbus 9
NL-6160 MA Geleen(NL)

(54) **Process and catalyst for the gas-phase isomerization of an alkene or cycloalkene oxide to the corresponding ketone.**

(57) Process and catalyst for the gasphase isomerization of a saturated alkene or cyclo alkene oxide to the corresponding ketone with a catalyst containing a noble metal of group VIII of the periodic system of elements deposited on a support and characterized in that the isomerization is effected in the pressure of hydrogen and in that a support having a basic character is applied.

EP 0 192 298 A1

# PROCESS AND CATALYST FOR THE GAS-PHASE ISOMERIZATION OF AN ALKENE OR CYCLOALKENE OXIDE TO THE CORRESPONDING KETONE

The invention relates to a process and a catalyst for the gas-phase isomerization of a saturated alkene or cycloalkene oxide to the corresponding ketone using a catalyst that contains a noble metal of group VIII of the periodic system of elements according to Mendeleev, and that is deposited on a support. Here and hereafter, saturated alkene or cycloalkene oxides shall be understood to mean, optionally substituted, saturated epoxides, which implies that unsaturated alkene oxides and aryl epoxides are excluded.

Such a process is described in US-A-2,799,708. Said patent relates to a catalytic isomerization of 2,3-epoxybutane and of saturated epoxides with 5-16 carbon atoms, the catalytically active metal being supported on an activated carbon base. Disadvantages of this known process are the low catalyst activity, which can be compensated only in part by applying a high temperature - (mention is made of a temperature between 300 and 430¤C), as well as the isomerization selectivity, which is low for a cycloalkene oxide (for the isomerization of 1,2-epoxycyclohexane a selectivity to cyclohexanone, at 100 % conversion, of 5 % is given). The patent also states that isomerization of a saturated epoxide with less than 5 C atoms mainly yields the aldehyde as reaction product.

The catalytic gas-phase isomerization of cyclohexene and methylcyclohexene oxides over solid acids and bases as catalyst is described, inter alia, by K. Arata et al. in Bull. Chem. Soc. Japan (1976) 49 563-4. This process, too, yields little cyclohexanone and, besides cyclohexanol, much cyclohexadiene.

The object of the invention is to provide a process as well as a catalyst for the gas-phase isomerization of a saturated alkene or cycloalkene oxide to the corresponding ketone with a high yield of ketone in comparison with other potential isomerization products such as the aldehyde or the unsaturated alcohol.

According to the invention, a process for the gas-phase isomerization of a saturated alkene or cycloalkene oxide to the corresponding ketone using a catalyst that contains a noble metal of group VIII of the periodic system of elements according to Mendelev and that is deposited on a support, is characterized in that the isomerization is effected in the presence of hydrogen and in that a support having a basic character is applied.

Surprisingly, it has been found that the presence of hydrogen is essential to the isomerization (without hydrogen being present there is hardly any catalytic activity), but that there is only a slight degree of hydrogenation to the corresponding saturated alcohol.

As regards the noble metal, a choice is preferably made from the group consisting of palladium and platinum, which combine good activity with high selectivity to the ketone. Use is made in particular of palladium, whose selectivity is higher.

It has been found that the presence of hydrogen is essential for good selective isomerization from an alkene or cycloalkene oxide to the corresponding ketone; in the absence of hydrogen there is hardly any isomerization, and replacement of hydrogen by nitrogen in an experiment results in rapid catalyst deactivation.

It is known that palladium obtained by reduction of, for instance, PdCl₂ with hydrogen is in a certain hydride form, depending on the temperature and the hydrogen partial pressure: the so-called α-palladiumhydride or β-palladiumhydride, as is described by A.J. Maeland et al. in J. Phys. Chem. 65 1270 (1961). It has now been found that the process according to the invention yields good results in particular when working in the range in which the palladium is in the α-palladiumhydride form.

The activity and the changes in activity can to a large degree be influenced by the choice of the catalyst support material. It has, for instance, been found that when the acidic γ-alumina is used as support, fast deactivation of the catalyst occurs, though in general it is known to apply strongly acid catalysts in the catalytic isomerization of alkene oxides to ketones. With respect to the isomerization of propylene oxide it is known that an increase in the acid strength of the support is attended by a shift in the isomerization selectivity from allylalcohol to propionaldehyde and finally to acetone (K. Arata et al., Catalysis Reviews, Sci. and Engng. 1983, 25(3) 383-5).

It has now, however, been found that addition of a base to the γ-alumina, particularly in the form of sodium carbonate, sodium hydrogen carbonate or sodium hydroxide, resulting in a conversion of the acid positions of the γ-alumina into neutral or preferably basic positions, has a surprising, positive effect on desactivation. When the support is given a basic character in this way, the deactivation rate decreases strongly. A sodium content of 0.5 to 5 % - (wt) has been found to yield special advantage when applying a palladium- or platinum-on-γ-alumina catalyst.

It is not only γ-alumina rendered basic that has been found to be suitable as a support for a catalyst for the gas-phase isomerization of an alkene or cycloalkene oxide. Support materials already having a basic character also are very suitable as support for a catalyst that can be used in the process according to the invention. By preference, for this purpose salts or oxides are chosen, the cation of the salt or oxide being taken from group IIA of the periodic system of elements according to Mendeleev. Particularly magnesium and calcium have been found to be suitable for this. The anion can be selected from the group consisting of oxide, hydroxide, carbonate and mixtures thereof. Preferably magnesium hydroxide is used as support material, since use of this support yields a strong decrease of the deactivation rate in comparison with the acid γ-Al₂O₃ referred to, while a high selectivity to the desired ketone is obtained.

By preference use is made of a catalyst that is given the form of a mantle catalyst, i.e. a catalyst with the noble metal preferentially applied to the outside of the support. Compared with a homogeneous catalyst, which is understood to mean a catalyst with the noble metal distributed throughout the pellet, this has the advantage that utilization of the noble metal is improved. As a result, the same catalytic behaviour is achieved using less noble metal.

The amount of noble metal used relative to the amount of support is not critical. Particularly suitable is a noble metal content of 0.5-5 % (wt) relative to the support.

The process according to the invention is suitable for the isomerization of both linear and cyclic, optionally substituted, saturated epoxides to the corresponding ketone. A non-exhaustive list of epoxides that can be isomerized to the corresponding ketone according to the process includes optionally substituted C₃-C₁₆ alkene oxides (such as propylene oxide, epichlorohydrin, butene oxide-2,3, butene oxide-1,2; 2,4-heptene oxide, cis 4,5-epoxyoctane and 1,2-epoxyoctane) as well as cyclohexene oxide and cyclododecene oxide and substituted derivatives thereof - (such as 1-methyl-cyclohexene oxide and 1,2-dimethylcyclohexene oxide).

The process temperature and pressure chosen are governed on the one hand by the fact that the component to be isomerized must be in gaseous condition under the reaction conditions, and on the other by the fact that at too high temperatures and/or reaction pressures the selectivity of the isomerization is lost by increasing hydrogenation of the components to be isomerized. A temperature of 415--525 K is very suitable, and preferably the temperature range of 450-500 K is used. The pressure is set so that the hydrogen partial pressure preferably is between 1 and 1000 kPa.

It has been found that a catalyst used in the process according to the invention exhibits deactivation, though only to a minor degree. To compensate for this decrease in activity, the temperature may be gradually increased during the isomerization process, but only to a limited degree, for at a too high temperature the compensation of the activity loss will be attended by a loss of isomerization selectivity because of increased hydrogenation of the alkene or cycloalkene oxide. It was tried to find a process in which the catalyst can be regenerated such that the original activity is wholly, or virtually wholly, restored while the selectivity is retained. Regeneration of a deactivated catalyst has been found possible by treatment with an oxygen--containing gas, preferably in the presence of steam.

Particularly suitable for this is an $N_2/O_2$ mixture. At the start of regeneration a low oxygen percentage in the gas is applied, viz. 1-5 vol.%, relative to $N_2$, which is gradually increased to, for instance, 20 vol.%. The temperature at which this regeneration is effected preferably is in the range of the 420-520 K. The amount of steam to be applied is not critical. Since steam affects the oxygen partial pressure in the regeneration gas, a large excess of steam will have an adverse effect on the regeneration speed. Preference is given to a steam - oxygen ratio from 1 : 1 to 10 : 1. After such a regeneration the catalyst can be reduced in a way known per se, upon which the original activity is wholly or virtually wholly restored. The rate at which a regenerated catalyst is desactivated appears to be almost the same as the rate at which a fresh catalyst is deactivated.

In applying the process according to the invention it has been found that, besides the isomerization of the alkene or cycloalkene oxide to the corresponding ketone, yet some degree of hydrogenation to the corresponding alcohol may occur. The selectivity of the process is such that the total selectivity to the ketone plus alcohol exceeds 90 %, while the ketone : alcohol ratio is at least 2 : 1 and is higher than or equal to 4 : 1 in a majority of cases, at a conversion of the alkene or cycloalkene oxide which is higher than or equal to 60 % and in the majority of cases even higher than or equal to 90 %.

The invention also relates to a catalyst that is suitable for the gas-phase isomerization of a saturated alkene or cycloalkene oxide to the corresponding ketone, which catalyst contains a noble metal of group VIII of the periodic system of the elements according to Mendeleev and is deposited on a support, the characterizing feature being that the noble metal is deposited on a support of basic material, consisting of a salt or an oxide, the cation of the salt or oxide being chosen from group IIA of the periodic system of the elements according to Mendeleev and the anion from the group formed by oxide, hydroxide, carbonate and mixtures thereof. Magnesium and calcium are particularly suitable as cation. The catalyst is suitable in particular when the noble metal is deposited such that a mantle catalyst is obtained, the noble metal preferentially being deposited on the outside of the support. Such a catalyst can, for instance, be obtained by impregnating a pre-formed basic support with a solution of a salt of the noble metal to be applied, following which the active mantle catalyst can be obtained, after washing and drying, by reduction with hydrogen as known per se.

The process will be elucidated with reference to the following, non-restrictive examples and comparative experiments.

The catalytic conversions of a saturated alkene or cycloalkene oxide are carried out in a vertical glass fixed--bed reactor (diameter 16 mm, length 20 cm) provided with a heating jacket. The reactor temperature is controlled by means of an oil thermostat. The catalyst bed (10-20 ml catalyst) is in the centre of the reactor tube. The space above and below the catalyst bed is filled with inert material (quartz fragments). Gases can be fed to the reactor, while the liquid alkene or cycloalkene oxide is pumped to the reactor top, where it evaporates on the inert material and is entrained over the catalyst bed by the gas stream. The reactor off-gas is cooled to 288 K and the liquid product is collected and analyzed gas chromatographically. The reduction of the catalysts takes place in the reactor during heating to the reaction temperature in a hydrogen steam. Unless stated otherwise, all experiments and examples were carried out at a total pressure of 100 kPa.

Comparative Example A

In the set-up described above 6 Nl/hour hydrogen and 10 ml/hour 1,2-epoxycyclohexane are passed over 20 ml 0.5 % $Ru/\gamma-Al_2O_3$ (supplied by Johnson Matthey) at a reactor temperature of 418 K. The changes with time in conversion and selectivities are given in Table 1. Besides cyclohexanol and cyclohexanone, as secondary products cyclohexane, cyclohexene and benzene are found.

TABLE 1

Isomerization of 1,2-epoxycyclohexane over a 0.5 % Ru/$\gamma$-Al$_2$O$_3$ catalyst.

Temperature 418 K        H$_2$/1,2-epoxycyclohexane : 2.7 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/ alcohol |
|---|---|---|---|---|---|
| h | % | mole % | mole % | mole % | mole/mole |
| 0.5 | 82.9 | 24.7 | 74.8 | 99.2 | 0.33 |
| 1 | 48.6 | 38.0 | 27.2 | 65.2 | 1.40 |
| 1.5 | 20.4 | 41.1 | 25.1 | 66.2 | 1.64 |
| 2 | 15.1 | 30.0 | 31.1 | 61.1 | 0.96 |
| 2.5 | 11.2 | 29.1 | 38.0 | 67.1 | 0.77 |

Comparative Example B

The reactor, filled with 20 ml 0.6 % Pt/$\gamma$-Al$_2$O$_3$ - (supplied by Engelhard), is charged with 6 Nl/hour hydrogen and 10 ml/h 1,2-epoxycyclohexane at a reaction temperature of 418 K. The changes with time in conversion and selectivity are shown in Table 2.

TABLE 2

Isomerization of 1,2-epoxycyclohexane over a 6 % Pt/$\gamma$-Al$_2$O$_3$ catalyst.

Temperature 418 K          H$_2$/1,2-epoxycyclohexane: 2.7 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/ alcohol |
|---|---|---|---|---|---|
| h | % | mole % | mole % | mole % | mole/mole |
| 0.75 | 88.1 | 16.6 | 81.8 | 99.4 | 0.20 |
| 1 | 85.1 | 9.9 | 88.6 | 98.5 | 0.11 |
| 1.5 | 75.5 | 11.6 | 84.3 | 95.9 | 0.14 |
| 2 | 63.1 | 13.0 | 85.0 | 98.0 | 0.15 |
| 2.5 | 50.3 | 15.2 | 81.0 | 96.2 | 0.19 |

Comparative Example C

To the reactor, which is filled with 20 ml 0.5 % Pd/$\gamma$-Al$_2$O$_3$ (Deoxo catalyst from Baker), 6 Nl/h hydrogen and 10 ml 1,2-epoxycyclohexane are added at a reaction temperature of 418 K. The changes with time in conversion and selectivities are shown in Table 3.

TABLE 3

Isomerization of 1,2-epoxycyclohexane over a 0.5 % Pd/γ-Al$_2$O$_3$ catalyst.

Temperature 418 K          H$_2$/1,2-epoxycyclohexane: 2.7 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/ alcohol |
|---|---|---|---|---|---|
| h | % | mole % | mole % | mole % | mole/mole |
| 0.25 | 99.9 | 10.0 | 89.7 | 99.7 | 0.11 |
| 0.83 | 85.9 | 35.6 | 63.9 | 99.5 | 0.56 |
| 1.5 | 47.1 | 51.3 | 48.1 | 99.4 | 1.07 |
| 2.75 | 40.0 | 51.5 | 47.5 | 99.0 | 1.08 |

Comparative experiments A through C show that the catalytic conversion of 1,2-epoxycyclohexane over catalysts containing a noble metal of group VIII of the periodic system according to Mendeleev under hydrogenating conditions leads to the formation of the isomerization product cyclohexanone, besides the expected formation of the hydrogenation product cyclohexanol. Because of a higher total selectivity to cyclohexanone and cyclohexanol, as well as a higher cyclohexanone : cyclohexanol ratio, palladium-containing catalysts are preferred.

Example I

The reactor, filled with 10 ml of a 1 % Pd/γ-Al$_2$O$_3$ catalyst containing 1-2 mass % Na (supplied by Engelhard), is charged with 3 Nl/h hydrogen and 5 ml/h 1,2-epoxycyclohexane. The reactor temperature is 418 K. After 3.5 hours operating time, the hydrogen is replaced by an equivalent amount of nitrogen.

From the changes in conversion and selectivities (see Table 4) the presence of hydrogen appears to be essential to the isomerization reaction. On replacement of hydrogen by nitrogen there is an abrupt decrease in the conversion of 1,2-epoxycyclohexane.

TABLE 4

Isomerization of 1,2-epoxycyclohexane over a 1 % Pd- 1.2 % Na/γ-Al2O3 catalyst.

Temperature 418 K          H2/1,2-epoxycyclohexane: 2.7 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/ alcohol |
|---|---|---|---|---|---|
| h | % | mole % | mole % | mole % | mole/mole |
| 1 | 93.8 | 44.2 | 55.3 | 99.8 | 0.80 |
| 1.5 | 90.8 | 51.3 | 48.2 | 99.5 | 1.06 |
| 2 | 88.6 | 54.6 | 44.9 | 99.5 | 1.22 |
| 3 | 82.8 | 59.1 | 40.5 | 99.6 | 1.46 |
| 3.5 | 77.0 | 62.3 | 37.1 | 99.4 | 1.68 |

Hydrogen replaced by equivalent amount of nitrogen

| | | | | | |
|---|---|---|---|---|---|
| 4 | 25.3 | 57.7 | 40.7 | 98.4 | 1.42 |
| 4.5 | 10.0 | 61.2 | 34.0 | 95.2 | 1.80 |
| 5 | 6.8 | 61.8 | 30.9 | 92.7 | 2.00 |

Example II

The reactor, which is filled with 20 ml 0.5 % Pd/γ-Al₂O₃ catalyst containing 0.8 mass % Na (supplied by Engelhard), is fed with 6 Nl/h hydrogen and 10 ml/h 1,2-epoxycyclohexane. The reactor temperature is 418 K. The changes with time in conversion and selectivities are presented in Table 5.

TABLE 5

Isomerization of 1,2-epoxycyclohexane over a 0.5 % Pd-0.8 % Na/$\gamma$-Al$_2$O$_3$ catalyst.

Temperature 418 K          H$_2$/1,2-epoxycyclohexane: 2.7 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/alcohol |
|---|---|---|---|---|---|
| h | % | mole % | mole % | mole % | mole/mole |
| 0.5 | 89.6 | 45.5 | 53.9 | 99.4 | 0.84 |
| 1 | 67.7 | 61.6 | 37.5 | 99.1 | 1.64 |
| 1.5 | 53.7 | 66.5 | 32.6 | 99.1 | 2.04 |
| 2 | 49.3 | 66.5 | 30.6 | 97.1 | 2.17 |
| 2.5 | 42.8 | 70.1 | 27.0 | 99.1 | 2.42 |
| 3 | 37.1 | 68.7 | 29.4 | 98.1 | 2.34 |
| 3.5 | 33.8 | 69.2 | 29.0 | 98.2 | 2.39 |
| 4 | 33.3 | 69.1 | 27.1 | 96.2 | 2.55 |
| 4.5 | 29.6 | 70.6 | 28.4 | 99.0 | 2.49 |

Example III

The reactor, filled with 20 ml 0.5 % Pd/$\gamma$-Al$_2$O$_3$ catalyst containing 1.2 mass % Na (supplied by Engelhard), is fed with 6 Nl/h hydrogen and 10 ml/h 1,2-epoxycyclohexane. The reactor temperature is 418 K. For the changes in conversion and selectivities, see Table 6.

TABLE 6

Isomerization of 1,2-epoxycyclohexane over a 0.5 % Pd-1.2 % Na/γ-Al₂O₃ catalyst.

Temperature 418 K              $H_2$/1,2-epoxycyclohexane: 2.7 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/ alcohol |
|---|---|---|---|---|---|
| h | % | mole % | mole % | mole % | mole/mole |
| 0.5 | 97.7 | 31.4 | 68.4 | 99.8 | 0.46 |
| 1 | 79.9 | 56.9 | 42.8 | 99.7 | 1.33 |
| 1.5 | 65.5 | 66.8 | 32.8 | 99.6 | 2.04 |
| 2 | 56.1 | 69.6 | 29.8 | 99.4 | 2.34 |
| 2.5 | 50.5 | 70.7 | 28.9 | 99.6 | 2.45 |
| 3 | 49.9 | 69.4 | 30.2 | 99.6 | 2.30 |
| 3.5 | 40.0 | 71.5 | 27.9 | 99.4 | 2.56 |
| 4 | 36.0 | 71.8 | 27.5 | 99.3 | 2.61 |
| 4.5 | 33.2 | 72.0 | 27.3 | 99.3 | 2.64 |

Comparison of Examples II and III shows that the catalyst with the highest alkali metal content deactivates slowest. The same favourable effect of the alkali metal is demonstrated in Examples IV and V for a 1 % Pd/γ-Al₂O₃ catalyst. The alkali metal can be deposited by the catalyst manufacturer during preparation of the catalyst, as was done with the catalysts of Examples II and III, but it can also be deposited on the finished catalyst.

Example IV

59 g of of a 1% Pd/γ-Al₂O₃ catalyst (supplied by Engelhard), is impregnated with 30 ml water in which 5.76 g sodium carbonate is dissolved. After this, the catalyst is dried, first on a steam bath and then in an oven at 393 K. The catalyst thus impregnated contains 5 mass % sodium.

The reactor, which is filled with 20 ml of this catalyst, is charged with 6 Nl/h hydrogen and 10 ml/h 1,2-epoxycyclohexane. The reactor temperature is 418 K. The changes with time in conversion and selectivities are given in Table 7.

TABLE 7

Isomerization of 1,2-epoxycyclohexane over a 1 % Pd-5 % Na/γ-Al$_2$O$_3$ catalyst.

Temperature 418 K　　　　　　　H$_2$/1,2-epoxycyclohexane: 2.7 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/ alcohol |
|---|---|---|---|---|---|
| h | % | mole % | mole % | mole % | mole/mole |
| 0.5 | 92.6 | 42.5 | 57.2 | 99.7 | 0.74 |
| 1 | 90.2 | 46.6 | 53.2 | 99.8 | 0.88 |
| 1.5 | 86.4 | 50.9 | 48.7 | 99.6 | 1.05 |
| 2 | 82.8 | 53.1 | 47.0 | 100 | 1.13 |
| 2.5 | 78.9 | 54.5 | 45.1 | 99.6 | 1.21 |
| 3 | 73.3 | 56.2 | 43.5 | 99.7 | 1.29 |
| 4 | 66.8 | 58.0 | 41.7 | 99.7 | 1.39 |
| 4.5 | 63.0 | 59.2 | 40.5 | 99.7 | 1.46 |
| 5 | 60.9 | 60.1 | 39.6 | 99.7 | 1.52 |

Example V

The reactor, filled with 20 ml 1 % Pd/γ-Al$_2$O$_3$ catalyst with a sodium content of 0.8 mass % (supplied by Engelhard), is charged with 6 Nl/h hydrogen and 10 ml/h 1,2-epoxycyclohexane. The reactor temperature is 418 K. The changes with time in conversion and selectivities are given in Table 8.

TABLE 8

Isomerization of 1,2-epoxycyclohexane over a 1 % Pd-0.8 % Na/$\gamma$-Al$_2$O$_3$ catalyst.

Temperature 418 K        H$_2$/1,2-epoxycyclohexane: 2.7 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/ alcohol |
|---|---|---|---|---|---|
| h | % | mole % | mole % | mole % | mole/mole |
| 0.5 | 96.3 | 7.5 | 92.2 | 99.7 | 0.08 |
| 1 | 99.4 | 24.1 | 75.6 | 99.7 | 0.32 |
| 1.5 | 92.1 | 47.8 | 51.8 | 99.6 | 0.92 |
| 2 | 82.4 | 58.4 | 41.3 | 99.7 | 1.41 |
| 2.5 | 73.8 | 62.2 | 37.7 | 99.9 | 1.65 |
| 3 | 68.5 | 63.9 | 35.6 | 99.5 | 1.79 |
| 3.5 | 64.1 | 64.8 | 34.7 | 99.5 | 1.87 |
| 4 | 55.7 | 66.0 | 34.0 | 100 | 1.94 |
| 4.5 | 53.0 | 66.8 | 32.7 | 99.5 | 2.04 |
| 5 | 46.5 | 66.3 | 33.0 | 99.3 | 2.01 |

Example VI

50 g of a 1 % Pd/$\gamma$-Al$_2$O$_3$ catalyst (supplied by Engelhard) is impregnated with 30 ml water in which 2.90 g sodium carbonate is dissolved. The catalyst is first dried on a steam bath and subsequently in an oven at 393 K. The catalyst thus impregnated contains 2.5 mass % sodium.

The reactor, which is filled with 20 ml of this catalyst, is charged with 6 Nl/h hydrogen and 10 ml/h 1,2-epoxycyclohexane. The reactor temperature is 453 K. The changes with time in conversion and selectivities are given in Table 9.

TABLE 9

## TABLE 9

Isomerization of 1,2-epoxycyclohexane over a 1 % Pd-2.5 % Na/$\gamma$-Al$_2$O$_3$ catalyst.

Temperature 453 K                    H$_2$/1,2-epoxycyclohexane: 2.7 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/alcohol |
|---|---|---|---|---|---|
| h | % | mole % | mole % | mole % | mole/mole |
| 0.5 | 99.0 | 30.6 | 69.1 | 99.7 | 0.44 |
| 1 | 97.1 | 41.8 | 57.9 | 99.7 | 0.72 |
| 1.5 | 94.3 | 49.7 | 49.9 | 99.6 | 1.00 |
| 2 | 90.1 | 55.8 | 44.6 | 100.4 | 1.25 |
| 2.5 | 86.6 | 60.0 | 39.7 | 99.7 | 1.51 |
| 3 | 81.3 | 64.1 | 35.5 | 99.6 | 1.81 |
| 3.5 | 76.7 | 67.1 | 32.6 | 99.7 | 2.06 |
| 5 | 70.1 | 70.3 | 29.0 | 99.3 | 2.42 |
| 5.5 | 64.6 | 73.1 | 26.4 | 99.5 | 2.77 |
| 6 | 62.5 | 73.8 | 25.6 | 99.4 | 2.88 |
| 6.5 | 61.1 | 74.4 | 24.9 | 99.3 | 2.99 |
| 7 | 59.0 | 76.1 | 23.5 | 99.6 | 3.24 |
| 7.5 | 58.5 | 75.2 | 23.8 | 99.0 | 3.16 |

Examples IV, V and VI show that catalyst deactivation proceeds slowlier at a higher alkali metal content of the catalyst. Comparison of Example V and II shows that a higher noble metal content also has a stabilizing effect.

Comparative experiment D

50 g zinc oxide (1/8 inch pellets, supplied by Harshaw) is socked in 50 ml water, after which 0.83 g palladium chloride and 1.70 g sodium chloride are added and the suspension is heated to the boiling point while being stirred. After 10 minutes the supernatant liquid is poured off and the catalyst is washed with 2 portions of 200 ml water. Subsequently, 19 g sodium hydrogen carbonate, dissolved in 150 ml water, is added and the mixture is heated to the boiling point. After this, 120 g methanol is added and the suspension is boiled for 15 minutes, the catalyst is filtered off, washed with 5 portions of 250 ml water and dried in an oven at 393 K. The palladium content of the catalyst thus obtained is 1 mass %.

The reactor, which is filled with 10 ml of the above-mentioned catalyst, is charged with 22.5 Nl/h hydrogen and 5 ml/h

1,2-epoxycyclohexane. The reactor temperature is 453 K. The changes with time in conversion and selectivities are given in Table 10.

TABLE 10

Isomerization of 1,2-epoxycyclohexane over a 1 % Pd/ZnO catalyst.

Temperature 453 K                    $H_2$/1,2-epoxycyclohexane: 19 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/ alcohol |
|---|---|---|---|---|---|
| h | % | mole % | mole % | mole % | mole/mole |
| 0.5 | 97.8 | 9.2 | 89.7 | 98.9 | 0.10 |
| 1 | 96.6 | 8.4 | 90.5 | 98.9 | 0.09 |
| 1.5 | 90.3 | 8.0 | 91.0 | 99.0 | 0.09 |
| 2 | 86.6 | 7.1 | 91.9 | 99.0 | 0.08 |
| 2.5 | 80.4 | 7.8 | 91.2 | 99.0 | 0.09 |
| 3.5 | 71.5 | 8.8 | 90.0 | 98.8 | 0.10 |
| 4 | 65.0 | 9.9 | 88.9 | 98.8 | 0.11 |
| 4.5 | 61.9 | 10.5 | 88.0 | 98.5 | 0.12 |
| 5 | 60.5 | 10.8 | 87.7 | 98.5 | 0.12 |
| 6 | 59.4 | 11.1 | 87.3 | 98.4 | 0.13 |

Example VII

50 mg magnesium oxide ("Leicht" grade, supplied by Lehmann und Voss) is mixed to a paste with 35 ml aqueous solution, which contains 120 g palladium oxide and 240 g sodium chloride per litre, use being made of a mechanical mixer. After curing of the catalyst paste, a hard mass is obtained, which is broken and screened to the desired particle size (2.4-3.4 mm). The catalyst thus prepared contains 5 mass % palladium. The palladium is distributed homogeneously dispersed throughout this catalyst.

The reactor, which is filled with 20 ml of this catalyst, is charged with 6 Nl/h hydrogen and 10 ml/h 1,2-epoxycyclohexane. The reactor temperature is 453 K. The changes with time in conversion and selectivities are given in Table 11.

TABLE 11

Isomerization of 1,2-epoxycyclohexane over a 5 % Pd/Mg(OH)₂ catalyst.

Temperature 453 K                    H₂/1,2-epoxycyclohexane: 2.7 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/ alcohol |
|---|---|---|---|---|---|
| h | % | mole % | mole % | mole % | mole/mole |
| 0.5 | 97.3 | 53.2 | 32.2 | 85.4 | 1.65 |
| 1 | 98.0 | 61.0 | 31.7 | 92.4 | 1.92 |
| 1.5 | 97.6 | 64.7 | 30.3 | 95.0 | 2.14 |
| 2 | 96.8 | 68.1 | 28.9 | 97.0 | 2.36 |
| 2.5 | 95.5 | 70.7 | 27.3 | 98.0 | 2.59 |
| 3.25 | 94.3 | 72.0 | 26.3 | 98.3 | 2.74 |
| 3.75 | 92.8 | 73.2 | 25.8 | 99.0 | 2.84 |
| 4.75 | 91.7 | 73.7 | 25.6 | 99.3 | 2.88 |
| 5.25 | 89.9 | 75.1 | 23.6 | 98.7 | 3.18 |
| 5.75 | 88.1 | 76.0 | 23.1 | 99.1 | 3.29 |
| 6.75 | 86.3 | 77.0 | 22.3 | 99.3 | 3.45 |
| 7.42 | 84.5 | 79.8 | 19.7 | 99.5 | 4.05 |

Comparative experiment D and Example VII show that the isomerization reaction is also catalyzed by palladium catalysts with an earth alkali metal oxide or hydroxide as support, in particular with magnesium hydroxide as support.

Example VIII

50 mg magnesium oxide ("Leicht" grade, supplied by Lehmann und Voss) is mixed with water until a paste is obtained. After curing at 120¤C, this paste is air-dried. After breaking, the magnesium hydroxide is screened to the 2.4-3.4 mm fraction. 25 mg magnesium hydroxide is impregnated with 20 ml solution of 40 g palladium chloride and 80 g sodium chloride per litre. This catalyst contains 2 mass % Pd; the palladium was found to be concentrated in the outer layer of the catalyst pellet.

The reactor, which is filled with 10 ml catalyst prepared in this way, is charged with 22.5 Nl/h hydrogen and 5 ml/h 1,2-epoxycyclohexane. The reactor temperature is 453 K. The changes with time in conversion and selectivities are presented in Table 12. From run 1 it is apparent that the conversion decreases from 100 % to 90 % in about 100 hours' time. The catalyst now is regenerated by passing over at 473 K of 100 NL/h of first nitrogen and then a nitrogen-oxygen mixture with an oxygen content that increases to 20 %. After regeneration, the catalyst is cooled to 298 K while nitrogen is passed over and then the catalyst is reduced using hydrogen. During the reduction, the temperature is slowly raised to the reaction temperature of 453 K, after which the second run is started. Table 12 shows that the original activity level is not fully reached when using this regeneration procedure and that the conversion dropped below 90 % in about half of the time of run 1.

After run 2, the catalyst again is regenerated. The reactor now is charged with 50 Nl/h hydrogen, 30 ml/h water and 6 NL/h air, the temperature being 493 K. The amount of air is slowly increased to 60 Nl/h, while the amount of nitrogen is simultaneously and proportionally reduced. After regeneration, the catalyst is reduced in the way described above. Table 12 (run 3) shows that the catalytic activity as well as the time it takes for the conversion to drop below 90 % are fully comparable to the values of run 1.

This example proves that full regeneration with an oxygen/nitrogen/ steam-containing gas mixture at temperatures below 500 K is possible and is to be preferred to regeneration with only an oxygen/nitrogen mixture.

TABLE 12

Isomerization of 1,2-epoxycyclohexane over a 2 % Pd/Mg(OH)$_2$ mantle catalyst.

Temperature 453 K        H$_2$/1,2-epoxycyclohexane: 19 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/ alcohol |
|---|---|---|---|---|---|
| h | % | mole % | mole % | mole % | mole/mole |
| run 1 | | | | | |
| 1 | 100 | 45.6 | 54.0 | 99.6 | 0.84 |
| 2 | 99.8 | 56.1 | 43.6 | 99.7 | 1.29 |
| 3 | 99.4 | 65.3 | 34.4 | 99.7 | 1.90 |
| 4 | 98.5 | 72.5 | 27.2 | 99.7 | 2.67 |
| 5 | 97.6 | 76.8 | 22.9 | 99.7 | 3.35 |
| 6 | 96.7 | 79.2 | 20.5 | 99.7 | 3.86 |
| 7 | 96.2 | 79.5 | 19.9 | 99.4 | 3.99 |
| 11 | 95.4 | 81.9 | 17.8 | 99.7 | 4.60 |
| 15 | 95.1 | 82.3 | 17.4 | 99.7 | 4.73 |
| 21 | 95.3 | 82.9 | 16.9 | 99.8 | 4.91 |
| 27 | 95.3 | 83.3 | 16.4 | 99.7 | 5.08 |
| 30 | 95.7 | 85.2 | 14.6 | 99.8 | 5.84 |
| 39 | 94.5 | 83.7 | 16.0 | 99.7 | 5.23 |
| 49 | 94.7 | 84.1 | 15.6 | 99.7 | 5.39 |
| 64 | 94.1 | 84.9 | 14.8 | 99.7 | 5.74 |

TABLE 12 (Continued)

Isomerization of 1,2-epoxycyclohexane over a 2 % Pd/Mg(OH)$_2$ mantle catalyst.

Temperature 453 K    H$_2$/1,2-epoxycyclohexane: 19 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/alcohol |
|---|---|---|---|---|---|
| h | % | mole % | mole % | mole % | mole/mole |
| 71 | 94.0 | 84.9 | 14.8 | 99.7 | 5.74 |
| 81 | 93.8 | 84.9 | 14.7 | 99.6 | 5.78 |
| 93 | 91.4 | 86.6 | 12.9 | 99.5 | 6.71 |
| 105 | 85.2 | 88.0 | 11.3 | 99.3 | 7.79 |

regeneration with oxygen/nitrogen mixture

run 2

| | | | | | |
|---|---|---|---|---|---|
| 1 | 90.3 | 37.9 | 61.9 | 99.8 | 0.61 |
| 2 | 98.8 | 67.0 | 32.7 | 99.7 | 2.05 |
| 4 | 97.6 | 76.7 | 22.9 | 99.6 | 3.35 |
| 8 | 94.8 | 82.0 | 17.5 | 99.5 | 4.69 |
| 12 | 92.6 | 84.4 | 15.2 | 99.6 | 5.55 |
| 19 | 91.2 | 86.5 | 13.1 | 99.6 | 6.60 |
| 28 | 90.4 | 86.6 | 13.0 | 99.6 | 6.66 |
| 36 | 89.4 | 87.4 | 12.3 | 99.7 | 7.11 |
| 44 | 89.2 | 87.6 | 12.0 | 99.6 | 7.30 |

TABLE 12 (Continued)

Isomerization of 1,2-epoxycyclohexane over a 2 % Pd/Mg(OH)$_2$ mantle catalyst.

Temperature 453 K          H$_2$/1,2-epoxycyclohexane: 19 moles/mole

| operating time | conversion of 1,2-epoxy-cyclohexane | selectivity to cyclo-hexanone | selectivity to cyclo-hexanol | total selectivity to cyclo-hexanone + cyclohexanol | ketone/ alcohol |
|---|---|---|---|---|---|
| h | % | mole % | mole % | mole % | mole/mole |

regeneration with oxygen/nitrogen/steam mixture

run 3

| | | | | | |
|---|---|---|---|---|---|
| 21 | 96.7 | 61.3 | 38.6 | 99.9 | 1.59 |
| 25 | 98.6 | 80.6 | 19.1 | 99.7 | 4.22 |
| 32 | 97.7 | 82.6 | 17.2 | 99.8 | 4.80 |
| 40 | 96.9 | 84.0 | 15.9 | 99.9 | 5.28 |
| 52 | 95.6 | 85.5 | 14.3 | 99.8 | 5.98 |
| 60 | 95.0 | 85.6 | 14.2 | 99.8 | 6.03 |
| 72 | 94.6 | 86.2 | 13.6 | 99.8 | 6.34 |
| 80 | 91.6 | 86.8 | 13.0 | 99.8 | 6.68 |
| 92 | 89.8 | 87.7 | 12.1 | 99.8 | 7.25 |
| 100 | 89.4 | 87.8 | 12.0 | 99.8 | 7.32 |

Example IX

The reactor is charged with 10 ml of the 2 % Pd/Mg-(OH)₂ mantle catalyst of Example VIII. Then a mixture of hydrogen and 1,2-epoxypropane is fed to the reactor under the conditions given in Table 13. The results presented in Table 14 show that the larger part of the 1,2 epoxypropane is converted into acetone.

Example X shows that, besides saturated cyclic alkene oxides, also non-cyclic saturated aliphatic alkene oxides can be converted into mainly the corresponding ketone in accordance with the process of the invention.

TABLE 13

Conversion of 1,2-epoxypropane over a 2 % Pd/Mg(OH)$_2$ mantle catalyst.

| operating time | temp. | additions epoxide | H$_2$ | H$_2$/epoxide | conversion | selectivity to acetone | selectivity to isopropanol |
|---|---|---|---|---|---|---|---|
| h | K | mL/h | NL/h | mole/mole | % | mole % | mole % |
| 0.5 | 443 | 10 | 22.5 | 6.7 | 31.0 | 81.7 | 11.9 |
| 1 | 453 | 10 | 22.5 | 6.7 | 59.6 | 73.2 | 19.3 |
| 2 | 453 | 10 | 22.5 | 6.7 | 62.9 | 71.1 | 21.1 |
| 3 | 543 | 10 | 11.0 | 3.3 | 53.5 | 83.6 | 9.2 |
| 4 | 563 | 10 | 11.0 | 3.3 | 58.8 | 83.8 | 8.0 |
| 5 | 463 | 5 | 22.5 | 13.3 | 60.4 | 86.1 | 7.3 |
| 6 | 463 | 5 | 22.5 | 13.3 | 60.5 | 86.9 | 6.8 |
| 7 | 463 | 5 | 22.5 | 13.3 | 63.9 | 86.8 | 6.7 |

**Claims**

1. Process for the gas-phase isomerization of a saturated alkene or cycloalkene oxide to the corresponding ketone using a catalyst that contains a noble metal of group VIII of the periodic system of elements according to Mendeleev and that is deposited on a support, characterized in that the isomerization is effected in the presence of hydrogen and that a support having a basic character is applied.

2. Process according to claim 1, characterized in that the support consists of γ-alumina which has been treated with a base so that the acid positions of the γ-alumina have been converted into neutral or, preferably, basic positions.

3. Process according to claim 1, characterized in that the support consists of a salt or oxide, the cation of the salt or oxide being taken from group II a of the periodic system of elements according to Mendeleev, and the anion being taken from the group formed by oxide, hydroxide, carbonate and mixtures thereof.

4. Process according to any one of claims 1-3, characterized in that the isomerization is effected at a temperature of 415-525 K.

5. Process according to claim 4, characterized in that the isomerization is effected at a temperature of 450-500 K.

6. Process according to any one of claims 1-5, characterized in that the isomerization is effected at a hydrogen partial pressure of 1-1000 kPa.

7. Process according to any one of claims 1-6, characterized in that the catalyst, deactivated through use in the isomerization, is regenerated by means of a treatment with an oxygen-containing gas.

8. Process according to claim 7, characterized in that a nitrogen-oxygen mixture is used as oxygen-containing gas.

9. Process according to any one of claims 7-8, characterized in that the regeneration is effected in the presence of steam.

10. Process according to claim 9, characterized in that the steam : oxygen ratio is between 1 : 1 and 10 : 1.

11. Catalyst suitable for the gas-phase isomerization of a saturated alkene or cycloalkene oxide to the corresponding ketone according to a process of any one of claims 1-10, the catalyst containing a noble metal of group VIII of the periodic system of elements according to Mendeleev and being deposited on a support, characterized in that the noble metal is deposited on a support of basic material consisting of a salt or oxide, the cation of the salt or oxide being taken from group IIA of the periodic system of elements according to Mendeleev, and the anion being taken from the group formed by oxide, hydroxide, carbonate and mixtures thereof.

12. Catalyst according to claim 11, characterized in that magnesium or calcium is used as cation.

13. Catalyst according to any one of claims 11-12, characterized in that magnesium hydroxide is used as support.

14. Catalyst according to any one of claims 11-13, characterized in that the noble metal is deposited on the support in such a way that a mantle catalyst is obtained.

15. Catalyst according to any one of claims 11-14, char-

acterized in that the noble metal content of the catalyst is 1-5 wt.%, calculated on the support.

European Patent
Office

**EUROPEAN SEARCH REPORT**

Application number

EP 86 20 0208

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 78, 1978, page 467, no. 57760n, Columbus, Ohio, US; & SU - A - 352 875 (S.I. KRYUKOV et al.) 29-09-1972 <br> --- | 1 | C 07 C 45/58 <br> C 07 C 49/403 <br> C 07 C 49/08 <br> B 01 J 23/40 |
| A | CHEMICAL ABSTRACTS, vol. 84, 1976, pages 508-509, no. 104787n, Columbus, Ohio, US; S. FUJII et al.: "Isomerization of propylene oxide over alkali cation X-zeolite catalysts", & WASEDA DAIGAKU RIKOGAKU KENKYUSHO HOKOKU 1974, 67, 26-9 <br> --- | 1 | |
| A | JOURNAL OF THE CHEMICAL SOCIETY, CHEM. COMM., no. 17, September 1979, pages 744-746; M. BARTOK et al.: "1,2-Bond shift isomerization of oxirans on noble metals" <br> * Page 745 * <br> --- | 1 | TECHNICAL FIELDS SEARCHED (Int. Cl.4) <br><br> C 07 C 45/00 <br> C 07 C 49/00 |
| D,A | BULLETIN OF THE CHEMICAL SOCIETY OF JAPAN, vol. 49, no. 2, February 1976, pages 563-564; K. ARATA et al.: "Epoxide rearrangement. IV. Isomerization of cyclohexene and 1-methylcyclohexene oxides over solid acids and bases in gas phase" <br> * Page 563 * <br> ----- | 1 | |

The present search report has been drawn up for all claims

| Place of search | Date of completion of the search | Examiner |
|---|---|---|
| THE HAGUE | 13-05-1986 | BONNEVALLE E.I.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82